# EUROPEAN PATENT APPLICATION

(11) **EP 2 666 487 A1**
(43) Date of publication of application: **27.11.2013**
(21) Application number: 12736680.5
(22) Date of filing: 10.01.2012
(51) Int. Cl.: A61M 1/00

(54) **DEVICE FOR EXTRACTING AND OBTAINING BLOOD PLASMA AND/OR FAT**

(30) Priority: 19.01.2011 ES 201100047
(71) Applicant: Jordà Ventosa, Enric, 08140 Caldes de Montbui (Barcelona) (ES)
(72) Inventor: Jordà Ventosa, Enric, 08140 Caldes de Montbui (Barcelona) (ES)
(74) Representative: Gallego Jiménez, José Fernando
(86) International application number: PCT/ES2012/000005
(87) International publication number: WO 2012/098273

(57) **Abstract**

The invention relates to a device for extracting and obtaining blood plasma and/or fat, comprising a syringe (1) formed from a hollow cylindrical body (2), with a luer-lock type (3) for coupling a needle (4) or a hermetic closing cap (5), and open on the opposite side, containing a plunger (6) that moves inside the body and has a hollow head (7) and a rod (8) that are brought together by means of screwing and can therefore be separated in the same way. The threaded rear end (9) is complementary to the threaded front end (10) of the rod (8). In its maximum extraction position, the head (7), together with its screwed rear zone (9), coincides with the end of the cylindrical body (2) of the syringe (1).

## Description

### OBJECT OF THE INVENTION

The invention, as expressed in the title of the present specification, relates to a device for extracting and obtaining blood plasma and/or fat, which has several novelty features that are inherent to its innovative construction and arrangement, which are described in detail below and, which provide advantages that are an improvement to the current state of the art in its scope.

### SCOPE OF THE INVENTION

The scope of the present invention falls within the technical sector of the industry involved in the manufacture of medical equipment and devices for extracting bodily fluids, particularly focusing on those for extracting blood plasma and fat.

### BACKGROUND OF THE INVENTION

At present, and referring to the state of the art, it should be noted that there are different systems for obtaining blood plasma on the market and, remarkable among these, due to being directly related to the object of the invention proposed herein, are the following:
- The one available from BTI Company (Biotechnology Institute) based on the pipettes made up of specific tubes for obtaining PRGF. These are inner disposable sterile tubes. They are equipped with a colour-coded safety cap with screw locking system and a measurement label to split and display the volume contained therein.
- The one called ART® and marketed by CURASAN, particularly intended for a healing method by means of the obtention of a platelet concentrate, which is essentially composed of a processor reservoir, several pre-filled syringes with different chemical solutions to be added to the blood, and several disposable syringes, as well as a cannula for extracting blood and corresponding adapters.
- The one marketed by Proteal and called Dispras, comprising a 20 ml multipurpose cylinder, with plunger and cap, and a 20 ml neutral cylinder, with a cap to compensate for weight during spinning, further using 20G needles, for infusion and venting of anticoagulated blood and syringes with luer-lock cone, connectors/transfers, sterility covers for the 20 ml cylinders, and aspiration cannula for the activator. This system works with a plunger, but needs an additional blood transfer step for the neutral cylinder and this may influence the aseptic environment.

- Finally the one designed by Arthrex company, which is based on a double syringe, by means of which the patient's blood extraction is carried out, which, without being replaced in another container, it is placed in the centrifuge, thus allowing, after the required time, plasma separation by simply pulling out the plunger of said syringe, which is itself a second syringe. This system skips said transfer step but only allows for extracting small amounts of 10 ml, since the configuration of the double syringe limits said capacity and also fails to separate the rich from the poor plasma.

Finally, it should be noted that there are other systems on the market, but they operate by filtration or decantation.

The aim of the present invention is therefore to develop a new device, which improves the effectiveness of the previously mentioned systems, and about which it can be assured that, at least, the applicant is not aware of the existence of any other device having similar technical, structural and constitutive features, the characterizing details thereof being conveniently collected in the appended claims that accompany the present specification.

### SUMMARY OF THE INVENTION

Specifically, what the invention proposes is a device intended to allow for extracting and obtaining blood plasma and/or fat, which is of the type using a syringe, by means of which the patient's blood extraction is carried out and, without replacing it in another container, it is placed in a centrifuge, thus allowing, after the required time, the separation of the plasma and/or fat, which will have been separated from other blood components therein.

To do this, the syringe consists of a right cylinder with a luer-lock-type thread at one end for coupling a needle or a cap, and a plunger, the rod of which has the particularity of being coupled to the head thereof by being screwed on, thus enabling separation between the two elements and separation of the head from the rod, thereby enabling the extraction and obtainment of a greater amount of plasma, since the total capacity of the syringe can be used as a container.

It should be noted that the dimension of the plunger head plus the thread thereof should coincide with the rear end of the outer cylinder which constitutes the syringe, once the maximum amount of fluid has been removed.

Thus, once the blood extraction has been carried out by means of said syringe, the rod is unscrewed from the plunger head and the syringe becomes a cylindrical container, suitable to be introduced directly into the centrifuge without having to transfer it to any pipette or any other container.

Subsequently, after the time necessary for the separation of plasma and/or fat, the cylinder shall be treated as if it were any pipette.

The described device for extracting and obtaining blood plasma and/or fat is therefore an innovative structure having hitherto unknown structural and constitutive features for said purpose, which together with its practical use, provide sufficient grounds to obtain the exclusivity privilege applied for.

### DESCRIPTION OF DRAWINGS

To complement the present description of the device of the invention and to assist in a better understanding of the features that distinguish it, a set of drawings is attached to the present specification as an integral part thereof, wherein, in an illustrative and non-limiting way, the following has been represented:

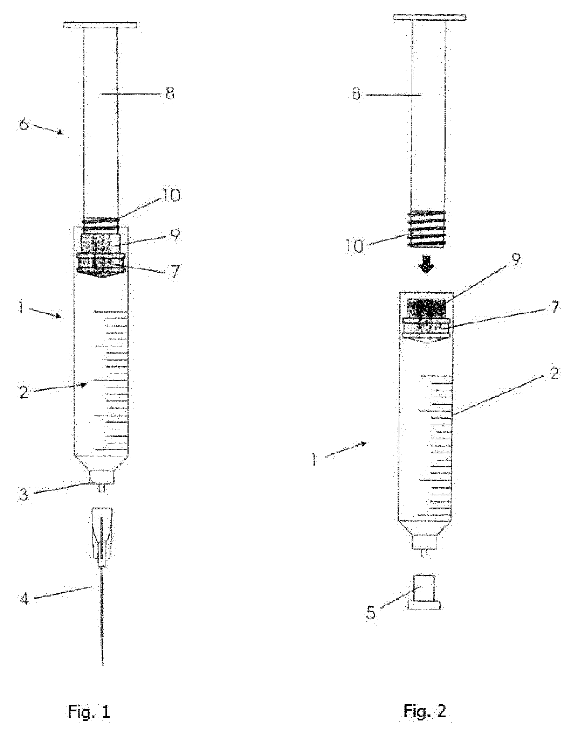

Figure 1. - Shows a sectional view of an embodiment example of the syringe which is the device for extracting and obtaining blood plasma and/or fat of the invention, which is shown as assembled in order to view the configuration and arrangement of the constituent parts and elements which characterize it.
Figure 2. - Shows a view similar to the previous one, but with the device elements separated.

### PREFERRED EMBODIMENT OF THE INVENTION

In view of the previously mentioned figures and according to the adopted numbering, a preferred embodiment example of the invention comprising the parts and elements, which are described in detail below, may be viewed.

Thus, as can be seen in these figures, the device in question is formed by a syringe (1) which, in a conventional manner, comprises a hollow cylindrical body (2), which is provided, at one end, with a luer-lock-type thread (3) for coupling a needle (4) or a hermetic closing cap (5) and is open on the opposite side, internally having a plunger (6) that moves inside it. Said plunger (6) has the particularity of consisting of a hollow head (7)and a rod (8) which are brought together by screwing and may, therefore, be separated from each other. To do this, said head (7) has a threaded rear end (9), which is complementary to the threaded front end (10) of the rod (8), so that said rod (8) may be unscrewed from the head (7).

It is important to note that, in its maximum extraction position, the head (7), together with its threaded rear end (9), coincides with the rear end of the cylindrical body(2) of the syringe (1).

Sufficiently described the nature of this invention, as well as how to implement it, it is not considered necessary to further explain to a person skilled in the art in order that he/she may understand the scope and advantages thereof, on the understanding that, in its essence, it may be implemented in other embodiments that differ in detail from that shown by way of example, and which shall also be covered by the claimed protection, provided that its fundamental principle is not changed or modified.

## Claims

1. Device for extracting and obtaining blood plasma and/or fat, of the type comprising a syringe (1), by means of which the patient's blood extraction is carried out and, without replacing it in another container, it is placed in a centrifuge, thus allowing, after the time required, the separation of plasma and/or fat; said syringe consisting of a hollow cylindrical body (2), with a luer-lock-type thread (3) for coupling a needle (4) or a hermetic closing cap (5) and open on the opposite end, internally having a plunger (6) that moves inside it, **characterized in that** said plunger (6) comprises a hollow head (7) and a rod(8) that are brought together by screwing and may therefore be separated from it.

2. Device for extracting and obtaining blood plasma and/or fat, according to claim 1, **characterized in that** the head (7) has a threaded rear end (9), which is complementary to the threaded front end (10) of the rod (8) so that the rod (8) may be unscrewed from the head (7) and **in that**, in its maximum extraction position, the head (7) together with its threaded rear end (9), coincides with the rear end of the cylindrical body (2) of the syringe (1).
